(19) Europäisches Patentamt
European Patent Office
Office européen des brevets

(11) Publication number: 0 339 982 A1

(12) **EUROPEAN PATENT APPLICATION**

(21) Application number: 89304174.9

(22) Date of filing: 26.04.89

(51) Int. Cl.[4]: **C 07 K 9/00**

(30) Priority: 28.04.88 GB 8810050
26.09.88 GB 8822510
16.02.89 GB 8903558

(43) Date of publication of application:
02.11.89 Bulletin 89/44

(84) Designated Contracting States:
AT BE CH DE ES FR GB GR IT LI LU NL SE

(71) Applicant: **BEECHAM GROUP PLC**
**Beecham House Great West Road**
**Brentford Middlesex TW8 9BD (GB)**

(72) Inventor: **Athalye, Medha Beecham Pharmaceuticals**
**Brockham Park Betchworth**
**Surrey RH3 7AJ (GB)**

**Coates, Nigel John Beecham Pharmaceuticals**
**Brockham Park Betchworth**
**Surrey RH3 7AJ (GB)**

**Milner, Peter Henry Beecham Pharmaceuticals**
**Brockham Park Betchworth**
**Surrey RH3 7AJ (GB)**

(74) Representative: **West, Vivien et al**
**Beecham Pharmaceuticals Great Burgh Yew Tree**
**Bottom Road**
**Epsom Surrey KT18 5XQ (GB)**

Claim for the following Contracting State: ES
The title of the invention has been amended (Guidelines for Examination in the EPO, A-III, 7.3).

(54) **Antibiotic compounds.**

(57) Glycopeptide antibiotics MM 47766, MM 47767, MM 55256 and MM 55260 are produced by Amycolatopsis orientalis strain NCIB 40011. Two of them (MM 47767 and MM 55256) are represented by the formula:

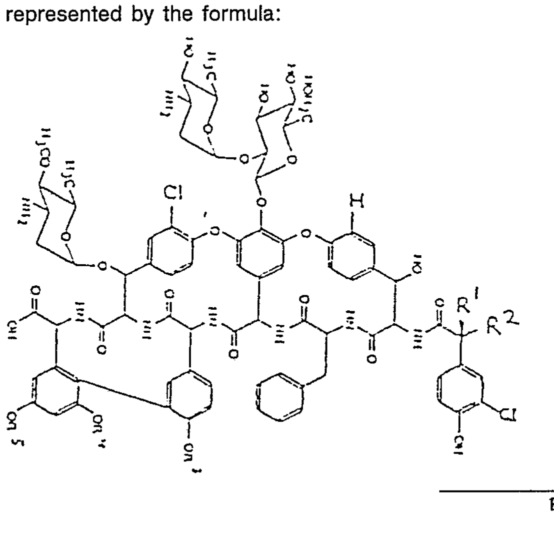

(I)

wherein $R^1$ is hydrogen and $R^2$ is methylamino (MM 47767) or $R^1$ is methylamino and $R^2$ is hydrogen (MM 55256) and wherein one of $R^3$, $R^4$ and $R^5$ is the group:

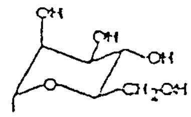

and the other two of $R^3$, $R^4$ and $R^5$ are hydrogen. The other two antibiotic (MM 47766 and MM 55269) are characterised through their physicochemical parameters. The aglycone MT 55261 and pseudoaglycone MT 55262 of MM 47767 also exhibit useful antibiotic activity.

EP 0 339 982 A1

## Description

# NOVEL COMPOUNDS

The present invention relates to novel antibacterially active materials, to processes for their production, to their pharmaceutical use, and to a novel microorganism from which they can be produced.

A large number of microorganisms have been isolated from nature and certain of those microorganisms have been found to produce various metabolites, which can be isolated and some of which have useful antibacterial activity. Four such metabolites are substances which have been designated MM 47766, MM 47767, MM 55256 and MM 55260. They are believed to be novel glycopeptide compounds and have been found to have useful antibacterial activity

The present invention accordingly provides the novel substances MM 47766, MM 47767, MM 55256 and MM 55260.

The present invention also provides a process for the production of the substance MM 47766, MM 47767, MM 55256 or MM 55260 which comprises cultivating a producing microorganism and subsequently isolating MM 47766, MM 47767, MM 55256 or MM 55260 or a derivative thereof from the culture.

The present invention furthermore provides a process for the preparation of the substance MM 47766, MM 47767, MM 55256 and/or MM 55260 which comprises separating MM 47766, MM 47767, MM 55256 and/or MM 55260 or a derivative thereof from a solution thereof in admixture with other antibacterially active substances and/or inactive substances by adsorption onto an affinity resin.

The substance MM 47766 has the following characteristics:

(i) it has an apparent molecular weight of 1969±2 by Fast Atom Bombardment (FAB) Mass Spectroscopy;

(ii) it may be obtained by the cultivation of a microorganism of the genus Amycolatopsis (previously known as Nocardia);

(iii) its retention time in high-performance liquid chromatography (h.p.l.c.), using a C18 μ Bondapak (Trade Mark) column packing (column size 3.9mm diameter x 300mm long), with an aqueous 0.1M $NaH_2PO_4$ solvent system at pH 6.0 containing 10% acetonitrile at a flow rate of 2ml/min, is approximately 4.6 minutes as measured by u.v. absorption at 220 and 280 nm(packed h.p.l.c. column supplied by Waters Associates, U.S.A.); and

(iv) it shows antibacterial activity against Staphylococcus aureus V573.

The substance MM 47767 has the following characteristics:

(i) it has an apparent molecular weight of 1807±2 by Fast Atom Bombardment (FAB) Mass Spectroscopy;

(ii) it may be obtained by the cultivation of a microorganism of the genus Amycolatopsis (previously known as Nocardia);

(iii) its retention time in high-performance liquid chromatography (h.p.l.c.), using a C18 μ Bondapak (Trade Mark) column packing (column size 3.9mm diameter x 300mm long), with an aqueous 0.1M $NaH_2PO_4$ solvent system at pH 6.0 containing 10% acetonitrile at a flow rate of 2ml/min, is approximately 7.4 minutes as measured by u.v. absorption at 220 and 280 nm(packed h.p.l.c. column supplied by Waters Associates, U.S.A.); and

(iv) it shows antibacterial activity against Staphylococcus aureus V573.

The substance MM 55256 has the following characteristics:

(i) it has an apparent molecular weight of 1807±2 by Fast Atom Bombardment (FAB) Mass Spectroscopy;

(ii) it may be obtained by the cultivation of a microorganism of the genus Amycolatopsis (previously known as Nocardia);

(iii) its retention time in high-performance liquid chromatography (H.P.L.C.), using a C18 μ Bondapak (Trade mark) column packing (column size 3.9mm diameter x 300mm long), with an aqueous 0.1M $NaH_2PO_4$ solvent system at pH 5.0 containing 15% acetonitrile and 0.005M sodium 1-heptanesulphonate ion pairing reagent at a flow rate of 2ml/min, is approximately 8.0 minutes as measured by u.v. absorption at 210 nm (packed h.p.l.c. column supplied by Waters Associates, U.S.A);

(iv) it is an epimer of MM 47767; and

(v) it shows antibacterial activity against Staphylococcus aureus V573.

The substance MM 55260 has the following characteristics:-

(i) it has an apparent molecular weight of 1807±1 by Fast Atom Bombardment (FAB) Mass Spectroscopy.

(ii) it may be obtained by the cultivation of a microorganism of the genus Amycolatopsis (previously known as Nocardia);

(iii) its retention time in high-performance liquid chromatography (HPLC) using a C18 μ Bondapak (Trade Mark) column packing (column size 3.9m diameter X 300mm long) with an aqueous 0.1m $NaH_2PO_4$ solvent system at pH 6.0 containing 10% $CH_3CN$ at a flow rate of 2ml/min. is approximately 33 minutes as measured by U.V. absorption at 220nm (packed HPLC column supplied by Waters Associates, U.S.A.).

(iv) it shows antibacterial activity against Staphylococcus aureus V573. MM 47767 and MM 55256 are believed to be the compounds of formula (I):

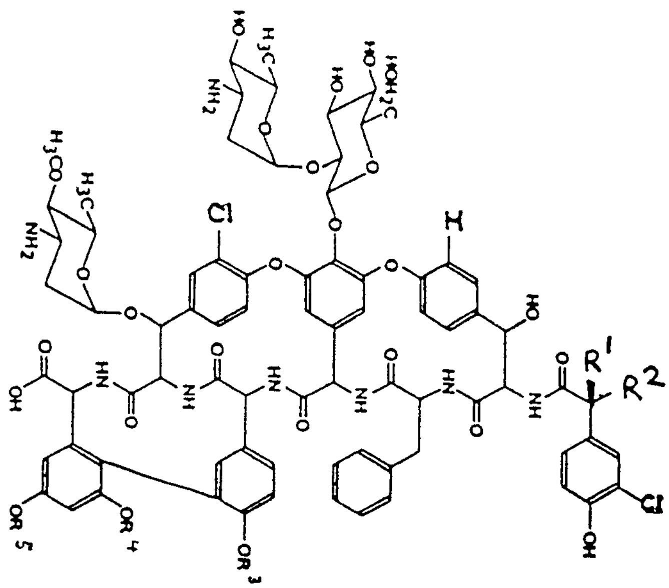

(I)

wherein $R^1$ is hydrogen and $R^2$ is methylamino (MM 47767) or $R^1$ is methylamino and $R^2$ is hydrogen (MM 55256). One of $R^3$, $R^4$ and $R^5$ is the sugar mannose and the other two of $R^3$, $R^4$ and $R^5$ are hydrogen.

The present invention also provides the aglycone and pseudoaglycone derivatives of MM 47767.

The aglycone, designated MT 55261 herein, has the formula (II).

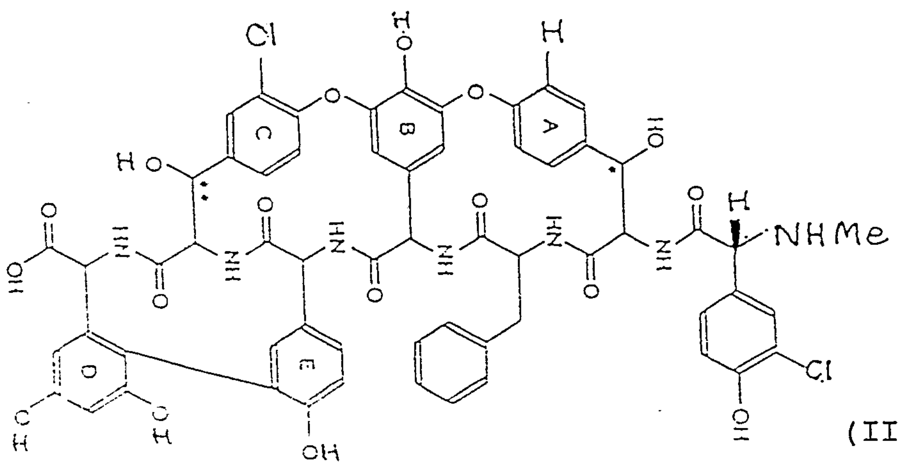

(II)

The pseudoaglycone, designated MT 55262 herein, has the formula (III).

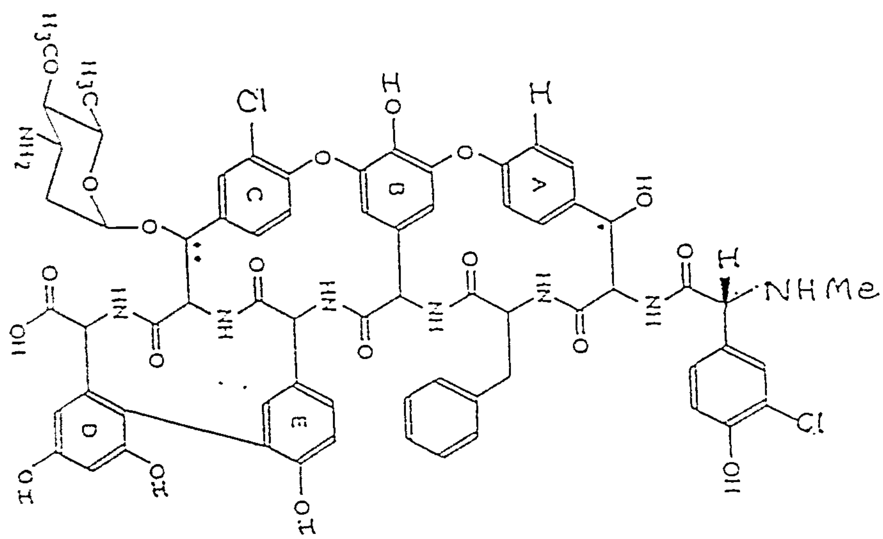

(III)

MM 47766, 47767, MM 55256 and MM 55260 may be obtained by the cultivation of a producing microorganism and the recovery of MM 47766, MM 47767, MM 55256 and/or MM 55260 or a derivative thereof from the culture.

The term 'cultivation' (and derivatives of that term) as used herein means the deliberate aerobic growth of an organism in the presence of assimilable sources of carbon, nitrogen, sulphur and mineral salts. Such aerobic growth may take place in a solid or semi-solid nutritive medium, or in a liquid medium in which the nutrients are dissolved or suspended. The cultivation may take place on an aerobic surface or by submerged culture. The nutritive medium may be composed of complex nutrients or may be chemically defined.

It has been found that suitable microorganisms for use in the cultivation process according to the invention include bacterial strains belonging to the genus Amycolatopsis (previously known as Nocardia) that are capable of elaborating MM 47766, MM 47767, MM 55256 and MM 55260. It has further been found that an example of such a strain is sp. NCIB 40011 and also mutants thereof, which has been isolated from nature.

The term 'mutant' as used herein includes any mutant strain which arises spontaneously or through the effect of an external agent whether that agent is applied deliberately or otherwise. Suitable methods of producing mutant strains including those outlined by H.I. Adler in 'Techniques for the Development of Microorganisms' in 'Radiation and Radioisotopes for Industrial Microorganisms', Proceedings of a Symposium, Vienna, 1973, page 241, International Atomic Energy Authority, and these include:

(i) Ionizing radiation (e.g. X-rays and λ-rays), u.v. light, u.v. light plus a photosensitizing agents (e.g. 8-methoxypsoralen), nitrous acid, hydroxylamine, pyrimidine base analogues (e.g. 5-bromouracil), acridines, alkylating agents (e.g. mustard gas, ethyl-methane sulphonate), hydrogen peroxide, phenols, formaldehyde, heat, and

(ii) Genetic techniques, including, for example, recombination, transformation, transduction, lysogenisation, lysogenic conversion, protoplast fusion and selective techniques for spontaneous mutants. Sp. NCIB 40011 has been identified as a previously unreported, atypical, strain of Amycolatopsis orientalis and therefore also forms a part of the present invention, particularly in biologically pure form. It has been deposited at the National Collections of Industrial and Marine Bacteria Ltd. (N.C.I.B), Aberdeen, Scotland under number 40011 on 11th April, 1988.

The fermentation medium for cultivating sp. NCIB 40011 suitably contains sources of assimilable carbon and assimilable nitrogen together with inorganic salts. Suitable sources of nitrogen include yeast extract, soyabean flour, meat extract, cottonseed, flour, malt, distillers dried solubles, amino acids, protein hydrolysates and ammonium and nitrate nitrogen. Suitable carbon sources include glucose, lactose, maltose, starch and glycerol. Suitably the culture medium also includes alkali metal ions (for example, sodium), halogen ions (for example, chloride), and alkaline earth metal ions (for example calcium and magnesium), as well as trace elements such as iron and cobalt.

The cultivation may suitably be effected at a temperature of about 20 to 35°C , advantageously 20 to 30°C, and the culture may suitably be harvested up to 7 days, advantageously about 3 to 5 days, after the initiation of fermentation in order to give an optimum yield of the desired product.

The desired product or a derivative thereof may then be isolated from the culture medium and worked up and purified using conventional techniques for glycopeptide compounds. All such isolation and purification

procedures may conveniently be effected at cool to ambient temperature, for example at a temperature within the range of from 4 to 30°C, conveniently from 20 to 25°C.

The desired product is generally obtained predominantly from the culture filtrate, and it is therefore convenient for the first isolation step to involve removal of solid material from the fermentation broth by, for example, filtration or centrifugation, to give a clarified culture filtrate.

Further isolation of the desired product from the clarified culture filtrate may conveniently be effected by adsorption onto an affinity resin such as D-alanyl-D-alanine-sepharose affinity resin.

The desired compound may readily be identified in a routine manner by testing for antibacterial activity and/or by monitoring the h.p.l.c. retention time.

Suitably, the separation procedure may include a high-performance liquid chromatography step, preferably as the last step. Elution may be effected using aqueous $NaH_2PO_4$/acetonitrile.

The aglycone and pseudoaglycone derivatives of MM 44767 may be prepared by hydrolysis, in particular acid hydrolysis, of MM 44767. In a preferred process MM 44767 is heated with a mineral acid such as hydrochloric acid and the process monitered by HPLC. In weak acid conditions (e.g. 1M HCl) heating for 10-15 minutes will produce the pseudoaglycone MT 55262. In stronger acid conditions (e.g. 5M HCl) more prolonged heating may produce the aglycone MT 55261.

MM 47766, MM 47767, MM 55256 and MM 55260 and their derivatives, in particular MT 55261 and MT 55262, may be crystalline or non-crystalline and, if crystalline, may optionally be hydrated or solvated.

The compounds according to the invention are suitably provided in substantially pure form, for example at least 50% pure, suitable at least 60% pure, advantageously at least 75% pure, preferably at least 85% pure, more preferably at least 95% pure, especially at least 98% pure, all percentages being calculated as weight/weight. An impure or less pure form of a compound according to the invention may, for example, be used in the preparation of a more pure form of the same compound or of a related compound (for example a corresponding derivative) suitable for pharmaceutical use.

MM 47766, MM 47767, MM 55256, MM 55260, MT 55261 and MT 55262 and their pharmaceutically acceptable derivatives have antibacterial properties and are useful for the treatment of bacterial infections in animals, especially mammals, including humans, in particular humans and domesticated animals (including farm animals). The compounds may be used for the treatment of infections caused by a wide range of organisms including, for example, those mentioned herein.

The present invention provides a pharmaceutical composition comprising MM 47766, MM 47767, MM 55256, MM 55260, MT 55261 or MT 55262 or a pharmaceutically acceptable derivative thereof together with a pharmaceutically acceptable carrier or excipient.

The present invention also provides a method of treating bacterial infections in animals, especially in humans and in domesticated mammals, which comprises administering MM 47766, MM 47767, MM 55256, MM 55260, MT 55261 or MT 55262 or a pharmaceutically acceptable derivative thereof, or a composition according to the invention, to a patient in need thereof.

The compounds and compositions according to the invention may be formulated for administration in any convenient way for use in human or veterinary medicine, by analogy with other antibiotics.

The compounds and compositions according to the invention may be formulated for administration by any route, for example oral, topical or parenteral. The compositions may, for example, be made up in the form of tablets, capsules, powders, granules, lozenges, creams, syrups, or liquid preparations, for example solutions or suspensions, which may be formulated for oral use or in sterile form for parenteral administration by injection or infusion.

Tablets and capsules for oral administration may be in unit dosage form, and may contain conventional excipients including, for example, binding agents, for example, syrup, acacia, gelatin, sorbitol, tragacanth, or polyvinylpyrrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or another suitable vehicle before use. Such liquid preparations may contain conventional additives, including, for example, suspending agents, for example sorbitol, methyl cellulose, glucose syrup, gelatin, hydroxyethyl cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and colour agents.

Compositions according to the invention intended for topical administration may, for example, be in the form of ointments, creams, lotions, eye ointments, eye drops, ear drops, impregnated dressings, and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

Compositions according to the invention may be formulated as suppositories, which may contain conventional suppository bases, for example cocoa-butter or other glycerides.

Compositions according to the invention intended for parenteral administration may conveniently be in fluid unit dosage forms, which may be prepared utilizing the compound and a sterile vehicle, water being preferred. The compound, depending on the vehicle and concentration used, may be either suspended or dissolved in the vehicle. In preparing solutions, the compound may be dissolved in water for injection and filter-sterilised before being filled into a suitable vial or ampoule, which is then sealed. Advantageously, conventional additives including, for example, local anaesthetics, preservatives, and buffering agents can be dissolved in the vehicle. In order to enhance the stability of the solution, the composition may be frozen after being filled into the vial, and the water removed under vacuum; the resulting dry lyophilized powder may then be sealed in the vial and a accompanying vial of water for injection may be supplied to reconstitute the liquid prior to use. Parenteral suspensions may be prepared in substantially the same manner except that the compound is suspended in the vehicle instead of being dissolved and sterilisation cannot be accomplished by filtration. The compound may instead be sterilised by exposure to ethylene oxide before being suspended in the sterile vehicle. Advantageously, a surfactant or wetting agent is included in such suspensions in order to facilitate uniform distribution of the compound.

A compound or composition according to the invention may suitable be administered to the patient in an antibacterially effective amount.

A composition according to the invention may suitably contain from 0.1% by weight, preferably from 10 to 60% by weight, of a compound according to the invention (based on the total weight of the composition), depending on the method of administration.

The compounds according to the invention may suitably be administered to the patient at a daily dosage of from 1.0 to 50 mg/kg of body weight. For an adult human (of approximately 70 kg body weight), from 50 to 3000 mg, for example about 1500 mg, of a compound according to the invention may be administered daily. Suitably, the dosage for adult humans is from 5 to 20 mg/kg per day. Higher or lower dosages may, however, be used in accordance with normal clinical practice.

When the compositions according to the invention are presented in unit dosage form, each unit dose may suitably comprise from 25 to 1000 mg, preferable from 50 to 500 mg, of a compound according to the invention.

The following Examples illustrate the present invention.

Production and Isolation of MM 47766 and MM 47767

Example 1

a) Fermentation

Culture NCIB 40011 was grown for 7 days at 26°C on a solid agar slant in a McCartney bottle. The agar medium had the following composition:

| Constituent | Amount (g/l) |
|---|---|
| Yeast extract | 4.0 |
| Malt extract | 10.0 |
| Dextrose | 4.0 |
| Agar | 20.0 |
| Deionised water | to 1 litre |

[The constituents were all 'Bacto' products (Bacto is a Trade Mark) as supplied by DIFCO Laboratories, P.O. Box 14B, Central Avenue, East Molesey, Surrey]. The medium was adjusted to pH 7.3 before sterilisation.

A spore suspension was prepared by adding 10ml of sterilised water containing 0.005% Triton X 100 to a Mccartney bottle agar culture of NCIB 40011, followed by sonication for 1 minute. Portions (1ml) of spore suspension were used to inoculate the fermentation medium (100ml) contained in 500ml conical flasks closed with foam plastic plugs. (Triton X 100 was obtained from B.D.H. Chemicals Ltd., Poole, Dorset). The fermentation medium contained:

| Constituent | Amount (g/l) |
|---|---|
| Soya bean flour | 10 |
| Glycerol | 20 |
| Maltose | 2 |
| Stock trace elements | 10ml |
| Deionised water | to 1 litre |

The stock trace element solution contained:

| Constituent | Amount (g/l) |
|---|---|
| $CaCl_2.2H_2O$ | 10 |
| $MgCl_2.6H_2O$ | 10 |
| NaCl | 10 |
| $FeCl_3$ | 3 |
| $ZnCl_2$ | 0.5 |
| $CuCl_2.2H_2O$ | 0.5 |
| $MnSO_4.4H_2O$ | 0.5 |
| $CoCl_2.6H_2O$ | 0.5 |

The medium was adjusted to pH 7.3 before sterilisation at 117°C for 15 minutes.

(The soya bean flour was Arkasoy 50 supplied by the British Arkady Co. Ltd., Old Trafford, Manchester).

Incubation of the fermentation flasks was carried out for 96 hours at 26°C and 240 rpm on a gyratory shaker. The harvested broth was then clarified by centrifugation. Samples were monitored for antibiotic activity by bioassay on Staphylococcus aureus 'Oxford' using the conventional hole-in-plate method.

b) Isolation of MM 47766 and MM 47767

The glycopeptides MM 47766 and MM 47767 were isolated from the clarified broth by adsorption onto D-alanyl-D-alanine-sepharose affinity resin.

The affinity adsorbent was prepared from D-alanyl-D-alanine immobilised on activated CH-sepharose 4B (6-Aminohexanoic acid-activated-sepharose-4B was obtained from Sigma Chemical Co., Poole, Dorset).

The clarified broth (1400ml) prepared as described in a) was stirred for 1 hour with D-alanyl-D-alaninesepharose affinity resin (14ml wet volume). The mixture was filtered onto a glass scinter funnel and the filtrate discarded. The affinity resin was resuspended in distilled water (1000ml) and filtered as before. The resin was washed once more with distilled water. The glycopeptides were eluted from the affinity resin with 50ml of 0.1M ammonia containing 50% acetonitrile. The eluate was evaporated under reduced pressure to dryness to yield 80mg of a mixture of MM 47766 and MM 47767.

This mixture was then redissolved in 1ml of distilled water and chromatographed on an HPLC column (9.4mm x 500mm) packed with Waters Preparative C18 reverse phase column packing (55-105 microns) (Waters Associates, 34 Maple Street, Milford, Mass. USA). The column was eluted with 0.1M $NaH_2PO_4$ pH 6.0 containing 10% acetonitrile, at a flow rate of 4ml/min. Fractions (8ml) were collected and bioassayed by disc diffusion on Staphylococcus aureus 'Oxford'. Antibacterially active fractions were also monitored on a Waters high performance liquid chromatography column (3.9 x 300mm) containing μbondapak C18 reverse phase material. The glycopeptides were eluted from the column with 0.1M $NaH_2PO_4$ pH 6.0 containing 10% acetonitrile, at a flow rate of 2ml/min. The eluate was monitored at 220nm and 280nm by a Hewlett-Packard 1040A diode array HPLC monitor (Hewlett-Packard, Corvallis Division, 100 N.E. Circle Boulevard, Corvallis, Oregon, USA). Under these conditions MM 47766 had a retention time of 4.6 minutes and MM 47767 had a retention time of 7.4 minutes (vancomycin standard has a retention time of 7.1 minutes under the same conditions). Fractions 6-8 contained MM 47766 and were combined (22ml). Fractions 10-15 contained MM 47767 and were combined (42ml).

Inorganic impurities were removed from the separate bulks by absorbing once more onto D-alanyl-D-alaninesepharose affinity resin (15ml Wet Volume per bulk). The resin was washed with water and the glycopeptide eluted with 0.1M ammonia containing 50% acetonitrile as previously described. The eluate in each case was evaporated to dryness to yield 2.3mg of MM 47766 and 9.8mg of MM 47767.

Properties of MM 47766

FAB mass spectroscopy indicated a molecular ion (MH+) at 1970±2.

Properties of MM 47767

FAB mass spectroscopy indicated a molecular ion (MH+) at 1808±2 and acid hydrolysis of the sample afforded phenylalanine and N-methyl m-chloro-p-hydroxyphenylglycine.

Molecular Formula: $C_{85}H_{95}N_9O_{31}Cl_2$

UV ($H_2O$) $\lambda_{max}$ 280nm ($\gamma$ 8028)

IR (KBr) 1653, 1605, 1595, 1501cm$^{-1}$

Figure 1 shows the 400MHz $^1$H NMR in DMSO $d_6$ at 353°k.

Tetramethylsilane as internal standard. N-methyl singlet occurs at $\delta$2.29.

The antibacterial activity of material produced essentially as in Example 1 was determined by the microtitre method. Oxoid No.2 broth (supplied by Oxoid Ltd, Wade Road, Basingstoke, Hampshire, UK (Oxoid is a trade mark)) was used for all organisms except for the Streptococcus spp. which was tested using Todd Hewitt broth (supplied by Oxoid Ltd.). Inocula were overnight broth cultures diluted tenfold. The microtitre plates were incubated for 24 hours at 37°C.

The results are shown in Table 1.

Table 1

Antibacterial activity of MM 47766 and MM 47767 against a range of organisms, determined by the microtitre method (MIC µg/ml)

| ORGANISM | MM 47766 | MM 47767 |
|---|---|---|
| Bacillus subtilis ATCC 6633 | 2.0 | 0.5 |
| Corynebacterium xerosis NCTC 9755 | 4.0 | 1.0 |
| Sarcina lutea NCTC 8340 | 8.0 | 2.0 |
| Staphylococcus aureus | | |
| Oxford | 8.0 | 4.0 |
| Russell | 8.0 | 8.0 |
| V573 MR* | 4.0 | 2.0 |
| S.saprophyticus 'FL1' | 32.0 | 8.0 |
| 'FL2' | 16.0 | 2.0 |
| S.epidermidis 60137 | 4.0 | 1.0 |
| 54815 | 32.0 | 32.0 |
| Streptococcus pyrogenes CN10 | 8.0 | 2.0 |
| 1950 | 4.0 | 0.5 |
| 1951 | 4.0 | $\leqq$0.5 |
| S.agalactiae 'Hester' | 4.0 | 1.0 |
| S.sanguis ATCC 10556 | 4.0 | 2.0 |
| S.viridans 'Harding' | 4.0 | $\leqq$0.5 |
| S.pneumoniae Pu7 | 4.0 | 1.0 |
| S.faecalis I | 8.0 | 2.0 |

* Multi-resistant (Methicillin, Tetracycline, Erythromycin and Gentamicin resistant)

Example 2: Preparation of MM 47767

a) Fermentation.

100ml of seed medium contained in a 500ml conicalflask fitted with a cotton gauze cap was sterilised at 121°C in an autoclave for 15 minutes. The flask was inoculated with 2ml of a spore suspension of culture NCIB 40011 which had been preserved in liquid nitrogen. The flask was then incubated at 26°C for 72hr on a gyratory shaking table at 240rpm. 10ml of the vegetative inoculum produced, was used to inoculate each of two further flasks of seed medium prepared in a similar way. Fermentation was then carried out for 48hr at 26°C on a gyratory shaking table at 240rpm. 15 litres of seed media together with 0.1% antifoaming agent, Polypropylene glycol P2000, was sterilised in a 20 litre fully baffled fermenter for 1 hour at 121°C. The fermenter was stirred by an agitator, fitted with three, vaned-disc impellers, at 200rpm and supplied with sterile air at 0.5 volumes per volume per minute. 200ml of the second seed stage were used to inoculate the fermenter and incubation was carried out for 48 hours at 26°C. An overpressure of air of 0.5 bar, was maintained throughout.

For the final fermentation, 300 litres of fermentation medium containing 0.1% P2000, was sterilised in a 450 litre fully baffled fermenter at 121°C for 1 hour. The fermenter was stirred with an agitator, fitted with three, vaned-disc impellers, at 50rpm. 8 litres of vegetative inoculum from the 20 litre fermenter were added and the fermentation incubated at 26°C for 72 hours. Sterile air was supplied at 0.25 volumes per volume per minute for

the first day and then increased to 0.5 v.v.m. for the remaining time. An overpressure of air of 0.5 bar was maintained throughout. The fermentation was harvested at 82hr and clarified by centrifugation.

The seed and fermentation media were of the same composition. The medium contained:

| Constituent | Amount (g/l) |
|---|---|
| Soyabean flour | 10 |
| Glycerol | 20 |
| Maltose | 2 |
| $CoCl_2.6H_2O$ | 0.005 |
| Trace element solution | 10ml |
| Deionised water | To 1 litre |

Trace element solution contained:

| Constituent | Amount (g/l) |
|---|---|
| $CaCl_2.2H_2O$ | 10 |
| $MgCl_2.6H_2O$ | 10 |
| NaCl | 10 |
| $FeCl_3$ | 3 |
| $ZnCl_2$ | 0.5 |
| $CuCl_2.2H_2O$ | 0.5 |
| $MnSO_4.4H_2O$ | 0.5 |

The medium was adjusted to pH 7.3 before sterilisation. (The soyabean flour was Arkasoy 50 supplied by Arkady - A.D.M., Manchester, U.K.)

b) Isolation of MM 47767

Clarified broth from Example 2a was further processed using an Alfa-Laval UFBR I/II Ultrafiltration rig (Alfa-Laval, Brentford, Middlesex) with Romicon hollow fibre cartridges. The ultrafiltrate (340 litres) was applied to a 22.6 litre Diaion HP20 column (HP20 supplied by Mitsubishi Chemical Industries, Tokyo, Japan) at a flow rate of 0.5 litre/min. The column was washed with 10 litres of water and then eluted with 50% propan-l-ol in 0.1M ammonia at 0.25 litre/min. 1 litre fractions were collected. Those with antibiotic activity (8-22) were bulked and evaporated under reduced pressure to 2.5 litres.

Precipitated solids were removed from the concentrate by centrifugation and the pellet washed twice with deionised water. The supernatant and the two water washes were combined (3.3 litres) and adjusted to pH 6.5 with HCl. This solution was then applied to a 0.75 litre CM Sephadex C25 cation exchange column ($Na^+$ form) previously equilibrated with 0.05M $NaH_2PO_4$ pH 6.5 (CM Sephadex was supplied by Pharmacia Ltd., Uppsala, Sweden). The column was washed with 0.05M $NaH_2PO_4$ pH 7.0 (0.9 litre) and both percolate and washings discarded. MM 47767 was eluted using an exponential gradient of 0.05M $NaH_2PO_4$ pH 7.0 to 0.2M $Na_2HPO_4$ pH 9.2 (1 litre in mixing vessel) at a flow rate of 10ml/min. 20ml fractions were collected, those containing MM 47767 (140-250) were bulked. The bulked fractions were applied to a 0.59 litre Diaion HP20 column at 14ml/min. The column was washed with 600ml of deionised water and eluted with 50% propan-1-ol in 0.1M ammonia. 20ml fractions were collected and those fractions containing MM 47767 (20-66) were bulked and concentrated *in vacuo*. $NaH_2PO_4$ was added to the concentrated solution to give a total of 1.5L with a molarity of 0.05M and the pH was adjusted to 6.5. The solution was then applied to a second CM Sephadex C25 cation exchange column (0.69 litre). The percolate was discarded and the column washed with 0.05M $NaH_2PO_4$ pH 7.0 (1 litre).

MM 47767 was eluted using an exponential gradient of 0.05M $NaH_2PO_4$ pH 7.0 to 0.2M $Na_2HPO_4$ pH 9.2 (1 litre in mixing vessel) at a flow rate of 10ml/min. 20ml fractions were collected, and those fractions containing MM 47767 (124-260) were bulked and desalted using D-alanyl-D-alanine Sepharose affinity resin. The resin was stirred with the bulked fractions for 1 hour at pH 7.0 and the resin recovered by filtration. The resin was then washed with distilled water (2 x 800ml aliquots) and eluted with six, 300ml aliquots of 0.1M ammonia containing 50% acetonitrile. The percolate and water washes which still contained some MM 47767 were treated once more with D-alanyl-D-alanine affinity resin and the resin recovered, washed and eluted as described above. The eluates containing MM 47767 were combined and the ammonia and acetonitrile evaporated off *in vacuo*. The solution was finally freeze-dried to yield 2.84g of substantially pure MM 47767.

Example 3

Preparation of MM 55256

MM 55256 can be isolated during the course of the isolation and extraction of MM 47767 as described in Example 2.

Following this procedure described in Example 2 MM 55256 can be separated from MM 47767 by its earlier elution from the first CM Sephadex column . Activity associated with MM 55256 was detected in fractions 82-136. These fractions were bulked and applied to a Diaion HP20 column (0.53 litre) at 10ml/min. The column was washed with 700ml of deionised water and eluted with 50% propan-1-ol in 0.1M ammonia. Fractions (20ml) were collected and those containing MM 55256 (3-21) were bulked and concentrated in vacuo. $NaH_2PO_4$ was added to the concentrated solution to give a total of 1.5 litres with a molarity of 0.05M and the pH adjusted to pH 6.5. The solution was then applied to a second CM Sephadex C25 cation exchange column (0.7 litre). The percolate was discarded and the column washed with 0.05M $NaH_2PO_4$ pH 7.0 (1.5 litres).

MM 55256 was eluted using an exponential gradient of 0.05M $NaH_2PO_4$ pH 7.0 to 0.2M $Na_2HPO_4$ pH 9.2 (1 litre in mixing vessel). Fractions (18ml) were collected and those fractions containing MM 55256 (80-145) were bulked and inorganic impurities removed by treating with D-alanyl-D-alanine sepharose affinity resin. The resin was stirred with the bulked fractions for 1 hour at pH 7.0 and the resin recovered by filtration. The resin was then washed with distilled water (2 x 1.5 litre aliquots) and eluted with 0.1M ammonia containing 50% acetonitrile (5 x 400ml aliquots). These were then concentrated in vacuo and freeze-dried to yield 757mg of MM 55256.

Example 4

A Method for Preparation of MM 55256 from MM 47767

A 30ml solution containing 100mg of MM 47767 prepared essentially as in Example 2 was heated at 70°C for 8 hours. Monitoring the resulting solution by ion-pair HPLC showed a mixture of MM 47767 and MM 55256 in the ratio of 45 : 55, (Waters C18 μBondapak column 3.8mm x 300mm eluting with 0.1M $NaH_2PO_4$ pH 5.0 + 15% $CH_3CN$ + 0.005M 1-heptanesulphonic acid sodium salt at a flow rate of 2ml/min, monitoring UV absorbance at 210nm) MM 47767 had a retention time of 5.4mins whilst MM 55256 had a retention time of 8mins (cf. vancomycin in this system has a retention time of 4.0mins).

$NaH_2PO_4$ was added to the above resulting solution to give a total of 200ml with a molarity of 0.005M at pH 6.5. The solution was then applied to a CM Sephadex C25 cation exchange column (182ml) packed in 0.05M $NaH_2PO_4$ pH 6.5. The percolate was discarded and the column washed with 0.05M $NaH_2PO_4$ pH 7.0 (500ml).

MM 55256 was eluted using an exponential gradient of 0.05M $NaH_2PO_4$ pH 7.0 to 0.2M $Na_2HPO_4$ pH 9.2 (500ml in mixing vessel). Fractions (16ml) were collected and those containing MM 55256 (28-43) were bulked and inorganic impurities removed using D-alanyl-D-alanine sepharose affinity resin. The resin was stirred with the bulked fractions for 1 hour at pH 7.0 and then recovered by filtration. The resin was then washed with deionised water (3 x 200ml aliquots) and eluted with 0.1M ammonia containing 50% acetonitrile (3 x 250ml aliquots). These were then bulked, concentrated in vacuo and freeze-dried to yield 35.6mg of MM 55256.

Physical and Spectroscopic Properties of MM 55256

Molecular Weight; 1807
Molecular Formula: $C_{85}H_{95}N_9O_{31}Cl_2$
400Mz $^1H$ NMR in DMSO at 353°K
Tetramethylsilane as internal standard.
Spectrum very similar to that of MM 47767 except for the N-methyl singlet which occurs at δ2.42.

Example 5

Preparation of MM 55260

A 5.7 litre portion of aqueous concentrate was obtained from the eluate of the first Diaion HP 20 column, prepared essentially as described in Example 2b.

The concentrate was adjusted to pH 6.5 by the addition of $Na_a H_2PO_4$ and after stirring at ambient temperature the resulting precipitate was removed by filtration.

The filtrate was applied to a 0.89 litre. CM Sephadex C25 cation exchange column, (Na+ form) previously equilibrated with 0.005 M $Na_a H_2 PO_4$ pH 6.5 (CM Sephadex was supplied by Pharmacia Ltd., Uppsala, Sweden). The column was washed with 0.005 M $Na_a H_2 PO_4$ pH 6.5 (0.85 litre) and both percolate and washings discarded.

The column was eluted using an exponential gradient of 0.05M $N_aH_2PO_4$ pH 6.8 to 0.2M $Na_2H PO_4$ pH 9.1 (1 litre in mixing vessel) at a flow rate of 20 ml/min. 20ml fractions were collected. Fractions 41-90 and 151-185 were bulked, (where 186-275 contained MM 47767 and 91-150 contained MM 55256). The bulked fractions were applied to a 1.4 litre Diaion HP 20 column at 60ml/min. The column was washed with 2 litres of deionised water and diluted with 2.5 litres of 50% propan-1-ol in 0.15M ammonia. The first 1 litre was discarded and the remaining 1.5 litres concentrated in vacuo.

$Na_a H_2PO_4$ was added to adjust the concentrated solution to pH6.5. The solution was then applied to a 0.98 litre CM Sephadex C25 cation exchange column equilibrated in 0.005M $N_aH_2PO_4$ pH 6.7. The column was washed with 200 ml of 0.005 M $N_aH_2PO_4$ pH 6.5 and 600 ml. of 0.05 M $N_aH_2PO_4$ pH 6.5. The percolate and washings were discarded and the column eluted using an exponential gradient of 0.05 M Na $H_2PO_4$ pH 6.5 to

0.1 M $Na_2$ $HPO_4$ pH 8.7 (1 litre in mixing vessel) at a flow rate of 20 ml/min. 20ml fractions were collected and monitored using UV absorbance at 280nm. Fractions 81-95 were bulked (where 166-210 contained MM 55256) and further processed on a Diaion HP 20 column (275 ml). After application of the bulked fractions, the column was washed with 500 ml of deionised water.

The percolate and washings were discarded and the column was eluted with 500 ml of 50% propan-1-ol in 0.15M ammonia.

After 160 ml, the eluate was collected and concentrated in vacuo.

This solution was treated with 25ml wet volume of D-alanyl-D-alanine Sepharose affinity resin by stirring for 1 hour at pH 7.0. The resin was recovered by filtration, washed with deionised water (2 x 50ml aliquots) and eluted with 0.1M ammonia containing 50% acetonitrile. (4x100 ml aliquots). The bulked eluates were concentrated in vacuo and freeze dried to yield 90 mg of product.

A 60 mg portion of this product was submitted to further chromatography using Matrex $C_{18}$ 20-45μ packing in a 22mm x 250mm glass column equilibrated in 0.1M $NaH_2$ $PO_4$ pH 6.0. (Matrex packing material was supplied by Amicon Ltd, Upper Mill, Stonehouse, Glos GL10 2BJ.)

The solid was dissolved in the equilibrating buffer to give 20 ml of solution which was applied to the column. The column was eluted with 0.1 M $NaH_2PO_4$ pH 6.0, containing 10% $CH_3CN$ at 3ml/min. 4.8 ml fractions were collected and monitored using U V absorbance at 280 nm. Fractions 116-145 were bulked, concentrated in vacuo and treated with D-alanyl-D-alanine Sepharose affinity resin, as previously described. The resulting concentrate was freeze dried to yield 13 mg of MM 55260. When submitted to HPLC (Waters C18 μ Bondapak column. 3.9 mm x 300 mm eluted with 0.1 M $NaH_2PO_4$ pH 6.0, containing 10% $CH_3CN$ at a flow rate of 2 ml/min, monitored by U V absorbance at 220 nm). MM 55260 had a retention time of 33 minutes, (cf. vancomycin in this system had a retention time of 7.1 minutes).

Properties of MM 55260

F A B mass spectroscopy indicated a molecular ion (M $Na^+$) at 1830±1.

## Example 6

Preparation of the aglycone of MM 47767 (MT 55261)

MM 47767 (100mg, 0.055 mmol) was dissolved in 5M hydrochloric acid (10 ml) and the resulting solution was heated in an oil bath at 100°C. The reaction was monitored by HPLC on a Spherisorb S10 ODS2 column eluting with acetonitrile/0.05M aqueous sodium acetate pH5.0 buffer mixtures. After 11 minutes the reaction solution was cooled in an ice bath and adjusted to pH 8.0 with dilute aqueous sodium hydroxide. The solution was then freeze dried and the resulting crude solid was de-salted and purified by passage through a column of Diaion HP20 SS resin eluting with water grading to 30% propan-1-ol in water. The fractions were monitored by HPLC (as above) and those containing the pure aglycone were pooled and freeze dried to afford the title compound, MT 55261 (51 mg, 76%). $\lambda_{max}(H_2O)$ 278nm (ε 7430); $\nu_{max}$ (KBr) 1653,1600,1507, 1209, 1061 $cm^{-1}$; m/z (positive xenon F.A.B; glycerol/thioglycerol/TFA) $MH^+$ 1212.

The antibacterial activity of MT 55261 was determined by the microtitre method as described in Example 1b. The results are shown in Table 2.

## Example 7

Preparation of the pseudoaglycone of MM 47767 (MT 55262)

MM 47767 (200mg, 0.11 mmol) in 1M hydrochloric acid (20ml) was heated in an oil bath at 100°C and the reaction solution was monitored by HPLC as described for the preparation of the corresponding aglycone, (Example 6). After 38 minutes the reaction solution was cooled in an ice bath and adjusted to pH 8.0 with dilute aqueous sodium hydroxide. The solution was then freeze dried and the resulting crude solid was de-salted and purified by passage through a column of Diaion HP20 SS resin eluting with water grading to 40% propan-1-ol in water. The fractions were monitored by HPLC (as above) and those containing the purified pseudoaglycone were pooled and freeze dried to afford the title compound, MT 55262 (35mg, 23%). $\lambda_{max}$ 277nm (ε 7565); $\nu_{max}$ (KBr) 1654, 1596,1490, 1211, 1060 $cm^{-1}$; m/z (positive xenon F.A.B; glycerol/thioglycerol/TFA) $MH^+$ 1355.

The antibacterial activity of MT 55262 was determined by the microtitre method as described in Example 1b. The results are shown in Table 2.

Table 2

Antibacterial activity of MT 55261 and MT 55262 against a range of organisms, determined by the microtitre method (MIC μg/ml)

| ORGANISM | MT 55261 | MT 55262 |
|---|---|---|
| Bacillus subtilis ATCC 6633 | 4.0 | 1.0 |
| Corynebacterium xerosis NCTC 9755 | - | - |
| Sarcina lutea NCTC 8340 | 8.0 | 1.0 |
| Staphylococcus aureus | | |
| Oxford | 8.0 | 1.0 |
| Russell | 8.0 | 4.0 |
| V573 MR* | 16 | 4.0 |
| S.saprophyticus 'FL1' | 16 | 4.0 |
| 'FL2' | 16 | 8.0 |
| S.epidermidis 60137 | 16 | 2.0 |
| 54815 | 16 | 4.0 |
| Streptococcus pyrogenes CN10 | 16 | 8.0 |
| 1950 | - | - |
| 1951 | 16 | 8.0 |
| S.agalactiae 'Hester' | 32 | 4.0 |
| S.sanguis ATCC 10556 | 32 | 16 |
| S.viridans 'Harding' | 32 | 4.0 |
| S.pneumoniae Pu7 | 32 | 4.0 |
| S.faecalis I | - | - |

* Multi-resistant (Methicillin, Tetracycline, Erythromycin and Gentamicin resistant)

Reference Example

Preparation of Affinity resin

The N-hydroxysuccinimide ester of 6-aminohexanoic acid sepharose 4B (60g) was placed on a glass sinter and washed with 1mM hydrochloric acid solution (2 litres) under suction. The wet cake was then added to a solution of D-alanyl-D-alanine (1.5g) in 0.1M sodium bicarbonate solution (60ml) and occasionally shaken over the next hour. The suspension was filtered under suction and the residue suspended in 0.1M tris (hydroxymethyl)aminomethane (TRIS) (100ml) for 1 hour and then refiltered through a glass sinter. The cake was washed successively with 0.1M sodium bicarbonate solution, 0.05M TRIS (containing 0.5M sodium chloride), 0.05M formate buffer at pH 4.0 (containing 0.5M sodium chloride) and finally distilled water. The affinity resin was then stored at 4°C in aqueous suspension.

**CLASSIFICATION OF NCIB 40011**

**Methods used**

The methods followed were those recommended by the International Streptomyces Project for the characterization of Streptomyces species [Shirling, E.B. and Gottlieb, D. "Methods for the characterization of

Streptomyces species" Int. J. Syst. Bacteriol.,16:313-340 (1966)] and those recommended for the characterization of Amycolata and Amycolatopsis species [Lechevalier, M.P., Prauser, H., Labeda, D.A. and Ruan, J.-S. "Two new genera of nocardioform actinomycetes: Amyolata gen. nov. and Amycolatopsis gen. nov." Int. J. Syst. Bacteriol. 36: 29-37 (1986)].

The isomers of diaminopimelic acid (DAP) and the carbohydrates in hydrolysates of whole cells were established by Thin Layer Chromatography (TLC) using the methods described by Komagata and Suzuki Komagata, K. and Suzuki, K.-I. "Lipid and Cell-Wall Analysis in Bacterial Systematics" Methods in Microbiology,19:161-207 (1987)]. Mycolic acids were determined by the methods described by Minnikin et al. [Minnikin, D. E., Hutchinson, I.G. and Caldicott, A.B. "Thin Layer Chromatography of Methanolysates of Mycolic Acid Containing Bacteria" J. Chromatogr. 188: 221-233 (1980)].

**RESULTS.**

**1. Cultural Characteristics:**

Culture NCIB 40011 grew on all the growth media recommended by the ISP, forming well developed, cream/pale orange to orange substrate mycelium and white aerial mycelium. No soluble pigments were detected on any of the media used. The cultural characterisitcs of NCIB 40011 on various media are summarised in Table 1.

**2. Chemical Characteristics:**

Hydrolysed whole-cells of the culture NCIB 40011 contained the meso isomer of diaminopimelic acid. Arabinose and Galactose were the major sugars present while ribose was detected in minor quantities. Mycolic acids were not present. These results indicate that culture NCIB 40011 has a cell-wall type IV with type A sugar pattern (Lechevalier et al, 1986). However, lack of mycolic acids places this culture firmly outside the genus Nocardia sensu stricto.

**3. Physiological characterisitcs:**

Key physiological characterisitcs of culture NCIB 40011 and Amycolatopsis orientalis ATCC 19795 are listed in Table 2.

Table 1′

Growth characteristics of NCIB 40011 after 14 days at 28°C

| MEDIUM | GROWTH | AERIAL MYCELIUM | | SUBSTRATE MYCELIUM | SOLUBLE PIGMENT |
|---|---|---|---|---|---|
| | | Formation | Colour | | |
| ISP 2 | Good | Poor | White | Pale orange | None |
| ISP 3 | Good | V poor | White | Orange | None |
| ISP 4 | Good | Fair | White | Pale orange | None |
| ISP 5 | Fair | Poor | White | Orange | None |
| ISP 6 | Poor | None | -- | Cream | None |
| ISP 7 | Good | Poor | Orange | Orange | None |
| Nutrient Agar | Poor | None | -- | Cream | None |
| Bennetts Agar | Good | None | -- | Cream | None |
| Czapek-Dox Agar | Good | V poor | White | Cream/white | None |

Table 2'

Physiological characterisitics of NCIB 40011 after 14 days at 28°C

| CHARACTERISTICS | NCIB 40011 | Amycolatopsis orientalis ATCC 19795 |
|---|---|---|
| Decomposition of: | | |
| Adenine | - | - |
| Casein | + | + |
| Hypoxanthine | - | + |
| Tyrosine | - | + |
| Xanthine | - | + |
| Production of: | | |
| Nitrate reductase | +/- | + |
| Amylase | + | - |
| Urease | + | + |
| Melanin | + | - |
| Esculinase | - | + |
| Gelatinase | - | + |
| Decarboxylation of: | | |
| Benzoate | - | - |
| Citrate | + | + |
| Mucate | - | - |
| Malate | + | + |
| Growth in the presence of: | | |
| Lysozyme (500 u./ml) | + | - |
| Salicylate | + | - |
| NaCl (5%, w/v) | + | + |
| Rifampicin (50 ug/ml) | - | + |
| Growth at: | | |
| 28 C | + | + |
| 37 C | + | - |
| 45 C | - | - |
| Utilization of carbohydrates as sole carbon sources: | | |
| Adonitol | - | + |
| Arabinose | - | + |
| Cellobiose | - | + |
| Dextrin | - | + |
| Erythritol | - | + |
| Galactose | + | + |
| Glucose | + | + |
| Inositol | + | + |
| Lactose | + | + |
| Maltose | - | + |
| Mannitol | + | + |
| Melibiose | - | + |
| $\alpha$-methyl-D-glucoside | - | + |
| Raffinose | - | - |
| Rhamnose | + | + |
| Salicin | - | + |
| Sorbitol | - | - |
| Sucrose | + | + |
| Trehalose | + | + |
| Xylose | - | + |
| (Control : no sugar) | - | - |

Key: +/- = variable result

**Identification of NCIB 40011:**

Based on key chemical, physiological and morphological features, culture NCIB 40011 is identified as an atypical strain of Amycolatopsis orientalis. The results obtained from physiological and morphological tests show that NCIB 40011 differs substantially from the published results for both the type strain of Amycolatopsis orientalis (ATCC 19795) and 21 other isolates of the species (Lechevalier et al., 1986). However, NCIB 40011 differs significantly from the published results for related genera such as Amycolatopsis orientalis subsp. lurida, Amycolatopsis mediterranea, Amycolatopsis rugosa and Amycolatopsis sulphurea (Lechevalier et al., 1986). Therefore, culture NCIB 40011 is identified as a new and atypical strain of the species Amycolatopsis orientalis.

## Claims

1. A compound of formula I or pharmaceutically acceptable derivative thereof:

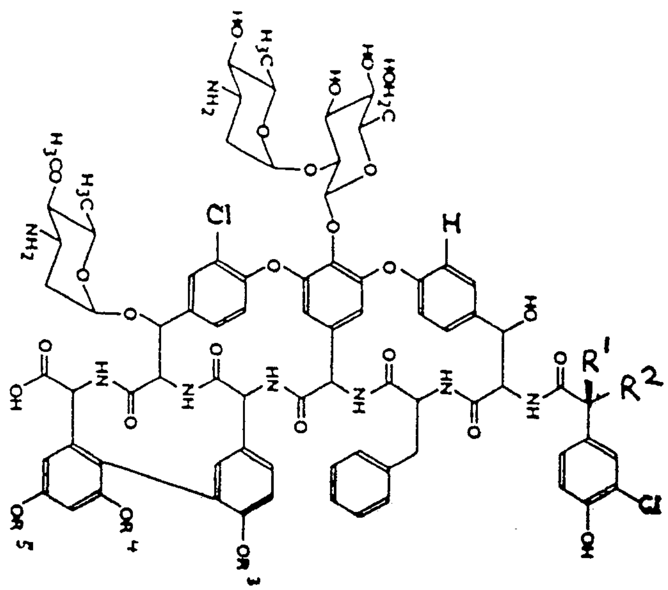

(I)

wherein $R^1$ is hydrogen and $R^2$ is methylamino (MM 47767) $R^1$ is methylamino and $R^2$ is hydrogen (MM 55256) and wherein one of $R^3$, $R^4$ and $R^5$ is the sugar mannose and the other two of $R^3$, $R^4$ and $R^5$ are hydrogen; or the substance designated MM 47766 characterised by having the following characteristics:-
(i) it has an apparent molecular weight of 1969±2 by Fast Atom Bombardment (FAB) Mass Spectroscopy;
(ii) it may be obtained by the cultivation of a microorganism of the genus Amycolatopsis (previously known as Nocardia);
(iii) its retention time in high-performance liquid chromatography (h.p.l.c.), using a C18 μ Bondapak (Trade Mark) column packing (column size 3.9mm diameter x 300mm long), with an aqueous 0.1M $N_aH_2PO_4$ solvent system at pH 6.0 containing 10% acetonitrile at a flow rate of 2ml/min, is approximately 4.6 minutes as measured by u.v. absorption at 220 and 280 nm(packed h.p.l.c. column supplied by Waters Associates, U.S.A.); and
(iv) it shows antibacterial activity against Staphylococcus aureus V573, or
the substance designated MM 55260 characterised by having the following characteristics:-
(i) it has an apparent molecular weight of 1807±1 by Fast Atom Bombardment (FAB) Mass Spectroscopy.
(ii) it may be obtained by the cultivation of a microorganism of the genus Amycolatopsis (previously known as Nocardia).
(iii) its retention time in high-performance liquid chromatography (HPLC) using a C18μ Bondapak (Trade Mark) column packing (column size 3.9mm diameter X 300mm long) with an aqueous 0.1m $N_aH_2PO_4$ solvent system at pH 6.0 containing 10% $CH_3CN$ at a flow rate of 2ml/min. is approximately 33 minutes as measured by U.V. absorption at 220nm (packed HPLC column supplied by Waters Associates, U.S.A.).
(iv) it shows antibacterial activity against Staphylococcus aureus V573.

2. A process for the production of MM 47766, MM 47767, MM 55256 or MM 55260 as defined in claim 1 which process comprises cultivating a producing micro-organism and subsequently isolating MM 47766, MM 47767, MM 55256 or MM 55260 or a derivative thereof from the culture.

3. A process for the preparation of the substance MM 47766, MM 47767, MM 55256 and/or MM 55260 which comprises separating MM 47766, MM 47767, MM 55256 and/or MM 55260 or a derivative thereof

from a solution thereof in admixture with other antibacterially active substances and/or inactive substances by adsorption onto an affinity resin.

4. A process as claimed in claim 2 or claim 3 wherein the producing microorganism is Amycolatopsis orientalis NCIB 40011.

5. Amycolatopsis orientalis NCIB 40011 or a mutant thereof in biologically pure form.

6. A compound designated MT 55261, of formula (II):

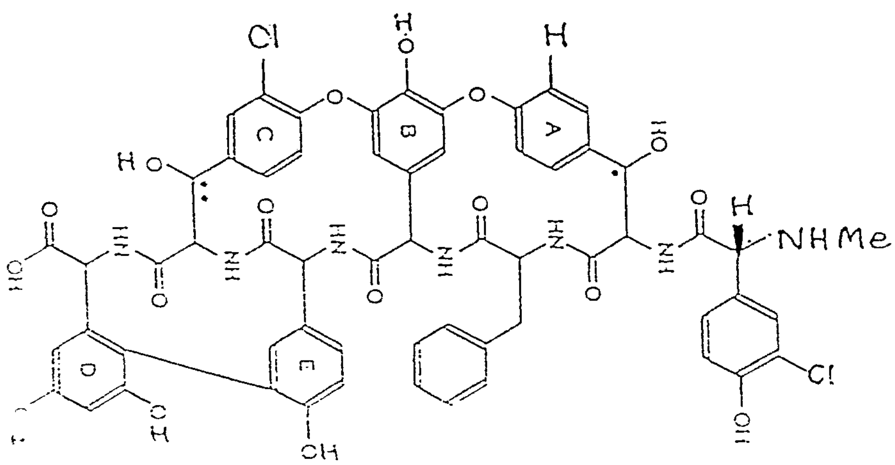

(II)

or a compound designated MT 55262 of formula (III):

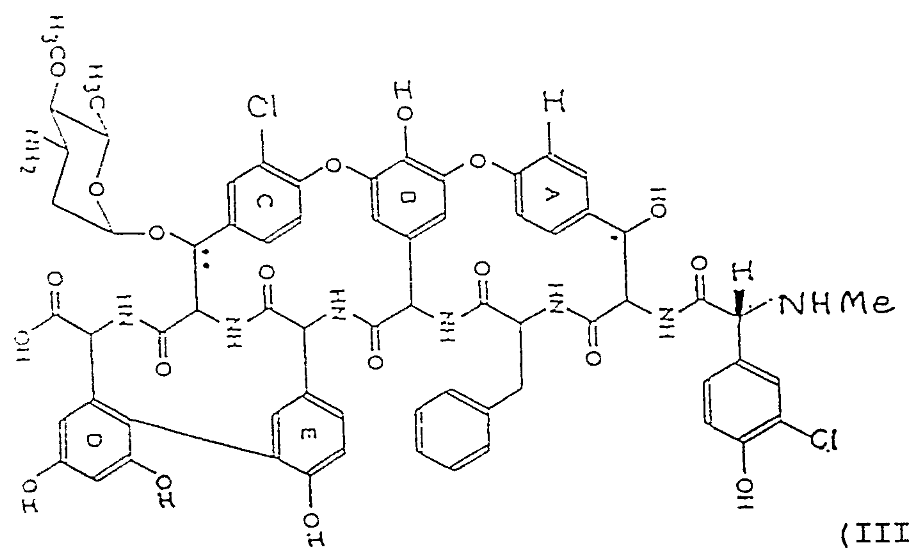

(III)

7. A process for the preparation of the compound MT 55261 of formula (II) or MT 55262 of formula (III) as defined in claim 6 comprising the hydrolysis of the compound MM 47767 of formula (I) as defined in claim 1.

8. A pharmaceutical composition comprising a compound according to claim 1 or a compound according to claim 6 together with a pharmaceutically acceptable carrier or excipient.

9. A method of treating bacterial infections in animals including humans which comprises administering thereto an effective non-toxic amount of a compound according to claim 1 or claim 6 or a composition according to claim 8.

10. A compound according to claim 1 or claim 6 for use in therapy.

11. A compound according to claim 1 or claim 6 for use in the treatment of bacterial infections in animals including humans.

12. Use of a compound according to claim 1 or claim 6 in the manufacture of a medicament for use in the treatment of bacterial infections in animals including humans.

**Claims for the following Contracting State: ES**

1. A process for the production of a compound of formula I or pharmaceutically acceptable derivative thereof:

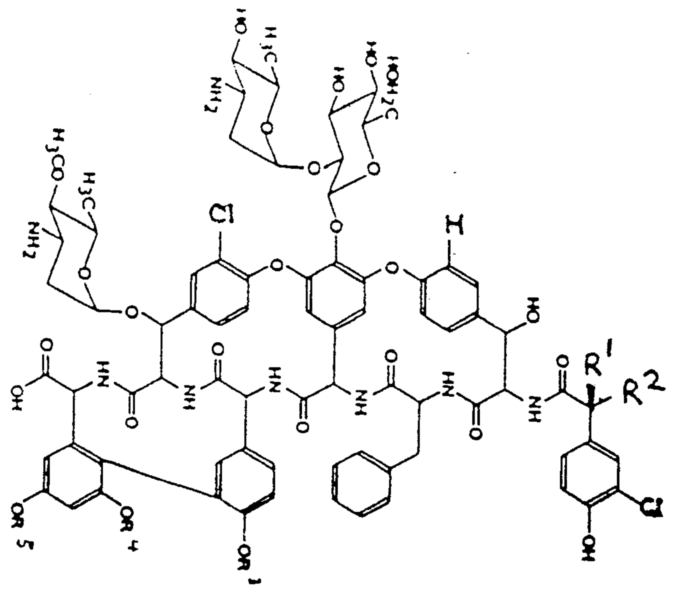

(I)

wherein $R^1$ is hydrogen and $R^2$ is methylamino (MM 47767) or $R^1$ is methylamino and $R^2$ is hydrogen (MM 55256) and wherein one of $R^3$, $R^4$ and $R^5$ is the sugar mannose and the other two of $R^3$, $R^4$ and $R^5$ are hydrogen; or the substance designated MM 47766 characterised by having the following characteristics:-
(i) it has an apparent molecular weight of 1969±2 by Fast Atom Bombardment (FAB) Mass Spectroscopy;
(ii) it may be obtained by the cultivation of a microorganism of the genus Amycolatopsis (previously known as Nocardia);
(iii) its retention time in high-performance liquid chromatography (h.p.l.c.), using a C18 μ Bondapak (Trade Mark) column packing (column size 3.9mm diameter x 300mm long), with an aqueous 0.1M $NaH_2PO_4$ solvent system at pH 6.0 containing 10% acetonitrile at a flow rate of 2ml/min, is approximately 4.6 minutes as measured by u.v. absorption at 220 and 280 nm(packed h.p.l.c. column supplied by Waters Associates, U.S.A.); and
(iv) it shows antibacterial activity against Staphylococcus aureus V573, or
the substance designated MM 55260 characterised by having the following characteristics:-
(i) it has an apparent molecular weight of 1807±1 by Fast Atom Bombardment (FAB) Mass Spectroscopy.
(ii) it may be obtained by the cultivation of a microorganism of the genus Amycolatopsis (previously known as Nocardia).
(iii) its retention time in high-performance liquid chromatography (HPLC) using a C18μ Bondapak (Trade Mark) column packing (column size 3.9mm diameter X 300mm long) with an aqueous 0.1m $N_aH_2PO_4$ solvent system at pH 6.0 containing 10% $CH_3CN$ at a flow rate of 2ml/min. is approximately 33 minutes as measured by U.V. absorption at 220nm (packed HPLC column supplied by Waters Associates, U.S.A.).
(iv) it shows antibacterial activity against Staphylococcus aureus V573,
which process comprises cultivating a producing micro-organism and subsequently isolating MM 47766, MM 47767, MM 55256 or MM 55260 or a derivative thereof from the culture.

2. A process for the preparation of the substance MM 47766, MM 47767, MM 55256 and/or MM 55260 which comprises separating MM 47766, MM 47767, MM 55256 and/or MM 55260 or a derivative thereof from a solution thereof in admixture with other antibacterially active substances and/or inactive substances by adsorption onto an affinity resin.

3. A process as claimed in claim 1 or claim 2 wherein the producing microorganism is Amycolatopsis orientalis NCIB 40011.

4. A process for the production of a compound, designated MT 55261, of formula (II):

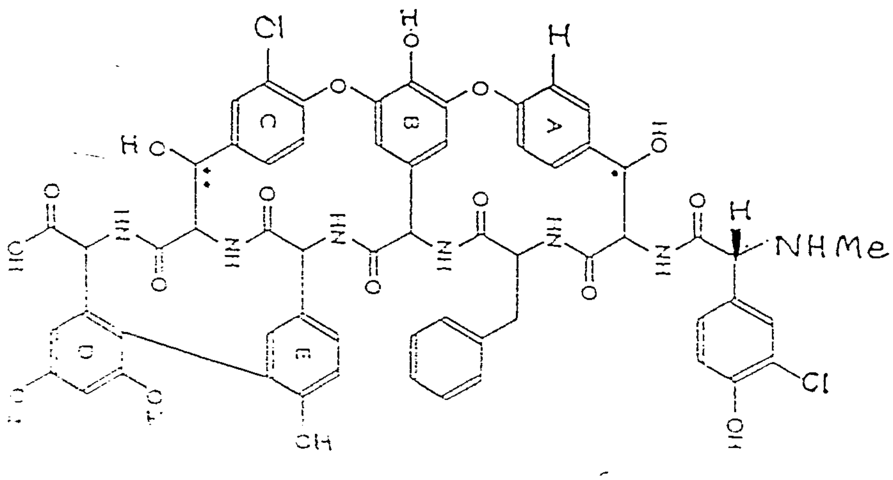

(II)

or a compound designated MT 55262 of formula (III):

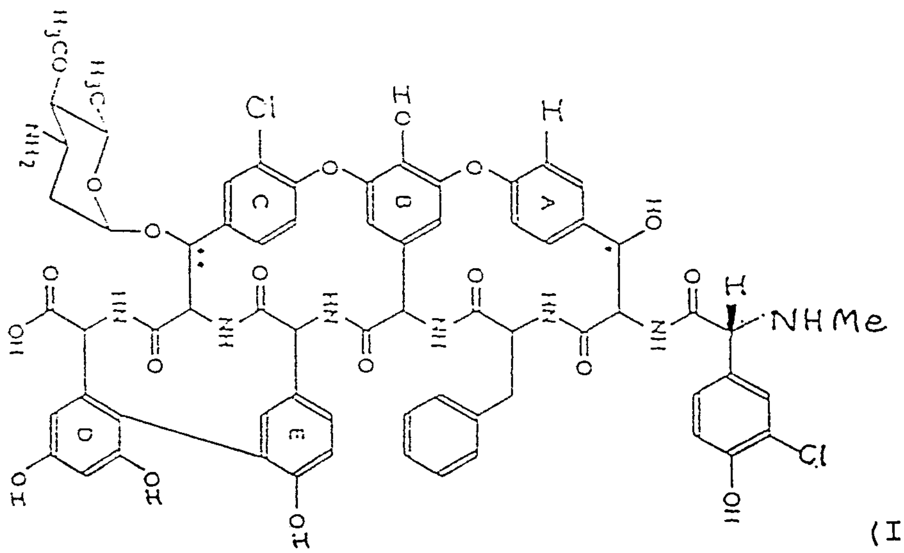

(III)

comprising the hydrolysis of the compound MM 47767 of formula I as defined in claim 1.

5. A process for the production of a pharmaceutical composition comprising admixing a compound MM 47766, MM 47767, MM 55256, MM 55200, MT 55261 or MT 55262 together with a pharmaceutically acceptable carrier or excipient.

6. A process according to claim 5 wherein from 0.1 to 60% by weight of the compound is admixed with the carrier or excipient.

7. A process according to claim 5 or claim 6 wherein the composition is formed into dosage forms for adminstration to the patient at a daily dosage of from 0.1 to 50 mg/kg of body weight.

8. Use of a compound MM 47766, MM 47767, MM 55256, MM 55260, MT 55261 or MT 55262 in the manufacture of a medicament for use in the treatment of bacterial infections in animals including humans.

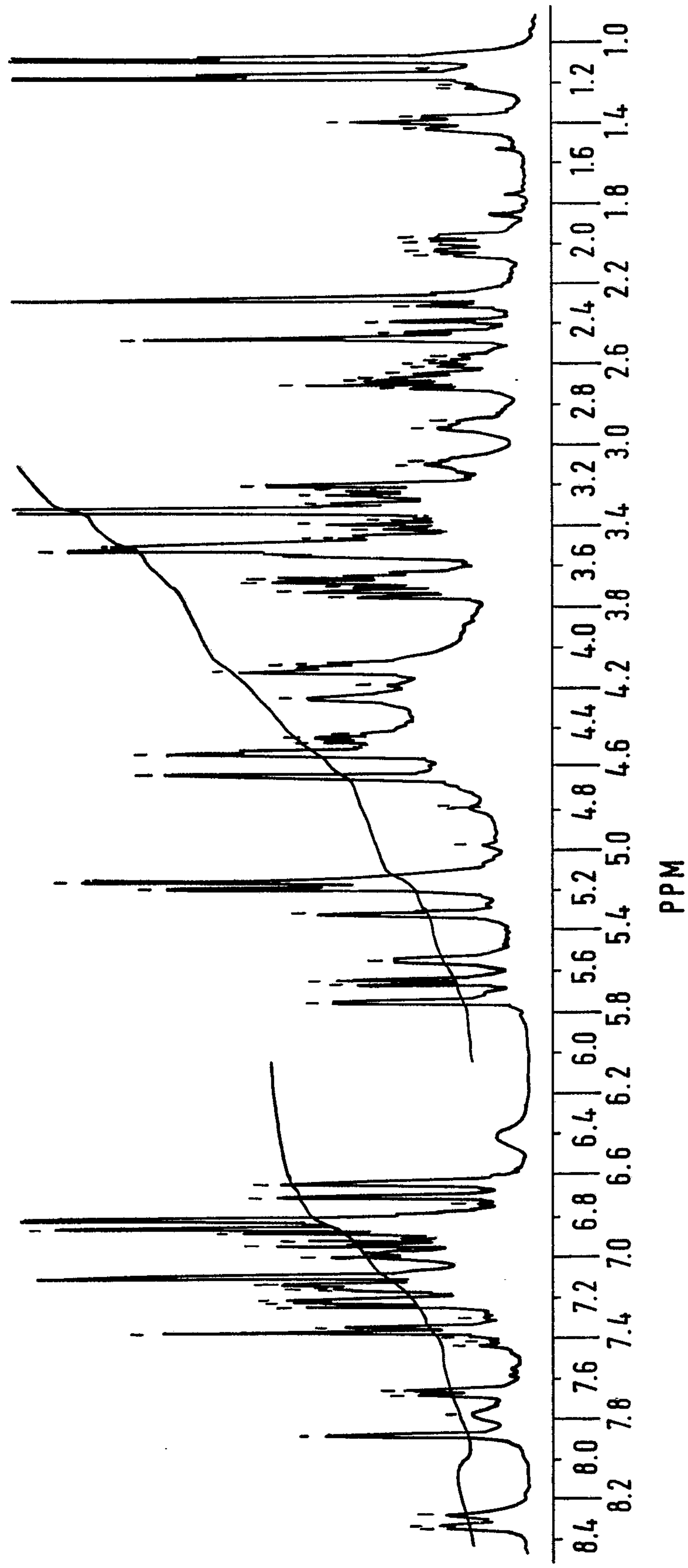


Fig. 1 ¹HNMR of MM 47767

European Patent Office

**PARTIAL EUROPEAN SEARCH REPORT**
which under Rule 45 of the European Patent Convention shall be considered, for the purposes of subsequent proceedings, as the European search report

Application number
EP 89 30 4174

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 255 299 (SMITHKLINE BECKMAN)<br>* Whole document *<br>-- | 1-8,10-12 | C 07 K 9/00 |
| A | EP-A- 0 265 143 (PFIZER)<br>* Whole document *<br>-------- | 1-8,10-12 | |

TECHNICAL FIELDS SEARCHED (Int. Cl.4)

C 07 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8,10-12
Claims searched incompletely:
Claims not searched: 9
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 12-06-1989 | MASTURZO |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82